# EUROPEAN PATENT SPECIFICATION

(11) **EP 2 799 049 B1**
(45) Date of publication and mention of the grant of the patent: **17.08.2016**
(21) Application number: 13755307.9
(22) Date of filing: 26.02.2013
(51) Int. Cl.: A61F 13/15, A61F 13/49

(54) **ABSORBENT ARTICLE MANUFACTURING DEVICE**
VERFAHREN ZUR HERSTELLUNG EINES SAUGFÄHIGEN ARTIKELS
DISPOSITIF DE FABRICATION D'ARTICLE ABSORBANT

(30) Priority: 29.02.2012 JP 2012042897
(43) Date of publication of application: 05.11.2014
(73) Proprietor: Unicharm Corporation, Ehime 799-0111 (JP)
(72) Inventor: ISHIKAWA, Shinichi, Kanonji-shi Kagawa 769-1602 (JP)
(74) Representative: Peter, Julian
(86) International application number: PCT/JP2013/054843
(87) International publication number: WO 2013/129348

(56) References cited:
- EP-A1- 1 415 628
- WO-A1-96/23470
- WO-A2-02/13744
- JP-A- H06 105 866
- JP-A- 2003 517 880
- JP-A- 2003 517 978
- JP-A- 2004 505 725
- JP-A- 2006 523 168
- JP-A- 2010 279 616
- US-A- 4 915 767

## Description

### [Technical Field]

The present invention relates to a manufacturing apparatus of an absorbent article with which an elastic member is arranged on a web which is continuous components of the absorbent article under conveyance.

### [Background Art]

Conventionally, in a manufacturing apparatus of an absorbent article such as a disposable diaper, in order to improve the fitting in the leg hole region of the wearer, a manufacturing step of arranging an elastic member at a position corresponding to the leg hole region is performed.

Specifically, in the manufacturing step, elastic members configuring leg gathers are arranged, in an elongated state and a curved shape, at a position corresponding to the leg hole region on a web which is continuous components forming one part of the absorbent article, and the absorbent article provided with leg gathers corresponding to the shape of the leg hole region is manufactured in continuity.

For example, Patent Literature 1 discloses a technique of arranging continuous elastic members configuring leg gathers on a web. A manufacturing apparatus according to Patent Literature 1 includes a plurality of rotating mechanism for conveying stripe-shaped web components as elastic members along the outer circumference. A rotating mechanism includes a web conveyor configured to convey the web components along the conveyance direction, and an applicator wheel configured to convey the web components supplied from the web conveyor in the conveyance direction while causing the web components to swing in the widthwise direction crossing the conveyance direction. The manufacturing apparatus according to Patent Literature 1 conveys the stripe-shaped web components in an elongated state, in the conveyance direction, while displacing the web components in the widthwise direction (see Fig. 4, Fig. 7, paragraph 0012, and paragraph 0017 of Patent Literature 1).

### [Related Art Document]

### [Patent Document]

### [Patent Document 1]

Japanese Unexamined Patent Application Publication No. 2004-505725 US 4,915,767 discloses an apparatus for applying an elastic ribbon onto the surface of a moving web in curved line path relative to the longitudinal centerline of the web. The apparatus includes a nip roll for directly rolling the elastic ribbon into engagement with the moving web and an oscillating roll adjacent to and spaced from the nip roll for oscillating the elastic ribbon in a direction transverse the centerline of the web and applying the elastic ribbon to the nip roll in the curved line path.

### [Summary of Invention]

Generally, a web component made from a stretchable material such as rubber and elastomer material, or from a composite web such as an elastic film and elastic nonwoven fabric is stretchable all over. In order to prevent such a web component from losing the characteristic as an elastic material, the web component is configured such that a stretching force cannot be applied at the distribution level and supply level. For example, the web component wound around a roller is wound in an almost relaxed state. A method of bonding such a web component in a relaxed state, onto a web such as a nonwoven fabric, in an elongated state, for example, includes changing a speed of the conveyance member configured to convey the web component, and then bonding the web component onto the web such as a nonwoven fabric, while elongating the web component.

For example, the manufacturing apparatus according to Patent Literature 1 is configured to convey a web component made from an elastomer onto an applicator wheel, in an elongated state. Generally, tensile stressing in the conveyance direction occur in an elastic member such as a web component, which is elongated in the conveyance direction, because of which in the state when the movement of the web component in the widthwise direction is not restricted, the width of the web component (length in the direction orthogonal to the conveyance direction) narrows down, that is, a so-called neck-in phenomenon occurs.

Particularly, as in the aforementioned method, when the web component is guided to the applicator wheel in an elongated state, the neck-in phenomenon tends to become marked if the conveyed distance in the state when the movement of the web component in the widthwise direction is not restricted becomes long before and after the web component is guided to the applicator wheel.

When the neck-in phenomenon takes place, stress occurs in the elastic member, such as the web component, along the widthwise direction due to narrowing of the width in the widthwise direction. The stretching state in the elastic members when stress occurs along the widthwise direction when the stress does not occur are different from the stretching state in the elastic members, even if the elastic members are elongated in the conveyance direction at the same elongation rate. Therefore, due to the occurrence of unwanted stress in the widthwise direction, the desired elastic manner might not be exhibited.

The present disclosure has been achieved in view of this condition, and an object thereof is to provide a manufacturing apparatus of an absorbent article with which it is possible to elongate and arrange stripe-shaped elastic members in a curved state while preventing the neck-in phenomenon.

In order to resolve the above problem, the manufacturing apparatus of an absorbent article manufacturing apparatus of an absorbent article 100) according to the present disclosure is summarized as an absorbent-article manufacturing apparatus in which stripe-shaped elastic members (leg hole elastic members 50) conveyed continuously are mounted on a web which is components of the absorbent article conveyed continuously, and which includes a first rotation mechanism (first rotation mechanism 111) configured to convey the elastic members along the outer circumference, with a first rotation axis (first rotation axis 111a) as the turning center, and a second rotation mechanism (second rotation mechanism 121) configured to convey the elastic members supplied from the first rotation mechanism along the outer circumference while displacing the elastic members in a widthwise direction (widthwise direction CD) orthogonal to the conveyance direction of the elastic members, with a second rotation axis (second rotation axis 121 a) as the turning center, such that the distance between the separation point (separation point P1) where the elastic members conveyed by the first rotation mechanism separate from the first rotation mechanism, and the contact point (contact point P2) where the elastic members first come in contact with the second rotation mechanism is shorter than the turning radius of at least one of the first rotation mechanism and the second rotation mechanism, the rotating velocity of the second rotation mechanism is faster than the rotating velocity of the first rotation mechanism, and the elastic members are elongated in the conveyance direction between the separation point and the contact point characterized in that the second rotation mechanism (121) is connected to a third rotation mechanism (122) configured to rotate about a third rotation axis (122a), and the second rotation mechanism (121) is configured to swing in the widthwise direction as a result of the third rotation mechanism (122), the elastic members (50) conveyed by the second rotation mechanism (121) are configured to be arranged on the web or to be sent out towards a guide mechanism (130, 230) while being displaced in the widthwise direction, while causing the leg hole elastic members (50) to swing in the widthwise direction in a predetermined cycle, and the axial direction of the third rotation axis (122a) is configured to cross the tangential direction where the elastic members (50) and the web or the guide member (130, 230) come into contact, and also cross the widthwise direction orthogonally.

### [Brief Description of Drawings]

[Fig. 1] Fig. 1 is a plan view of an absorbent article according to an embodiment.
[Fig. 2] Fig. 2 is a side view illustrating a manufacturing apparatus of an absorbent article according to the embodiment.
[Fig. 3] Fig. 3 is an expanded view of a second rotation mechanism and a third rotation mechanism portions illustrated in Fig. 2.
[Fig. 4] Fig. 4 is a plan view schematically illustrating the elastic manner of an elastic member.
[Fig. 5] Fig. 5 is a cross-sectional view schematically illustrating the elastic manner of an elastic member.
[Fig. 6] Fig. 6 is a diagram schematically illustrating an swinging mechanism portion configured to displace the elastic member in the widthwise direction.
[Fig. 7] Fig. 7 is a side view of a manufacturing apparatus of an absorbent article according to a modification.

### [Description of Embodiments]

Next, a manufacturing apparatus of an absorbent article according to the present invention is explained with reference to drawings. Specifically, (1) Configuration of absorbent article, (2) Method of manufacturing absorbent article, (3) Configuration of manufacturing apparatus of an absorbent article, (4) Elongation manner of leg hole elastic members, (5) Swinging manner of leg hole elastic members, (6) Manufacturing apparatus of an absorbent article according to modification, and (7) Other embodiments are explained.

In the following description of the drawings, the same or similar reference numerals are used to designate the same or similar parts. It will be appreciated that the drawings are schematically shown and the ratio and the like of each dimension are different from the real ones. Therefore, a specific dimension should be determined in view of the following description. Of course, among the drawings, the dimensional relationship and the ratio may be different.

### (1) Configuration of absorbent article

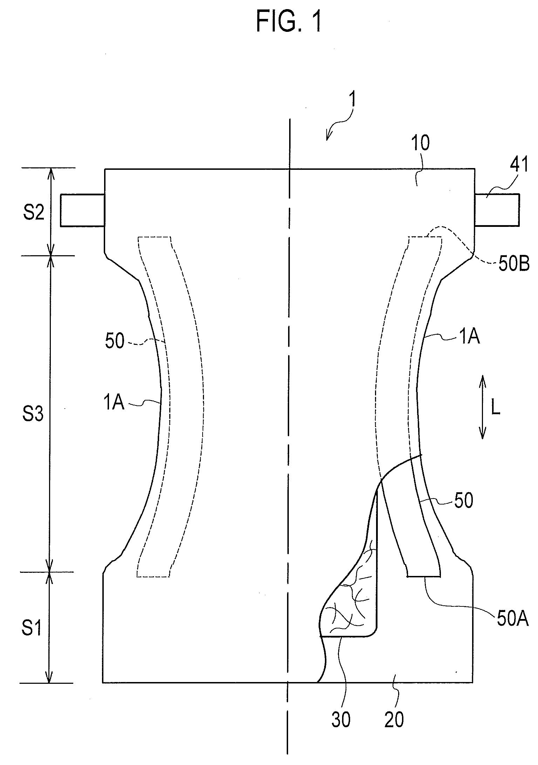

First of all, a configuration of an absorbent article 1 according to the present embodiment is explained with reference to drawings. Fig. 1 is a plan view showing the absorbent article 1 according to the present embodiment. In the present embodiment, the absorbent article 1 is an open-type disposable diaper. Fig. 1 is an exploded plan view of the state when the leg hole elastic member is elongated.

In the present embodiment, the open-type diaper is defined as not a diaper that is already formed in the shape of pants, but defined as a diaper in an expanded state before use, and worn by the wearer by fastening predetermined portions of the diaper with a fastening tape or the like. It should be noted that the present embodiment is configured to manufacture an open-type disposable diaper; however, the present embodiment may also be configured to manufacture a pant-type disposable diaper.

The absorbent article 1 has a front waistline region S1 corresponding to the front waist of the wearer, a back waistline region S2 corresponding to the back waistline of the wearer, and a crotch region S3 corresponding to the crotch of the wearer, between the front waistline region S1 and the back waistline region S2, in the longitudinal direction L of the absorbent article 1. The absorbent article 1 includes a topsheet 10, an exterior sheet 20, and an absorber 30.

The topsheet 10 is provided at the side that is in contact with the skin of the wearer. The topsheet 10 is formed of a liquid-permeable sheet such as a hydrophilic nonwoven fabric or woven cloth, an aperture plastic film, and an aperture hydrophobic nonwoven fabric.

The exterior sheet 20 includes a back nonwoven fabric that is in contact with the clothing, and a liquid-impermeable film (hereinafter, called the back film) positioned towards a skin contact surface side from the back nonwoven fabric and formed of a water-resistive film (for example, polyethylene). The back film is made of a moisture-permeable or moisture-impermeable film.

The absorber 30 is provided between the topsheet 10 and the exterior sheet 20. The absorber 30 is configured to absorb the excretions, such as the bodily fluid, of the wearer. The absorber 30 includes an absorbent core formed of pulp and superabsorbent polymer, and an absorbent sheet, such as a tissue, to cover the absorbent core.

A side portion 1A, which is the end in the widthwise direction of the topsheet 10 and the exterior sheet 20, recesses towards the inside of the widthwise direction in the crotch region S3. The region surrounding the recess of the crotch region S3 is the leg hole region arranged along the leg of the wearer. In the leg hole region, a pair of leg hole elastic members 50 that form the leg hole gathers are arranged.

The leg hole elastic members 50 are in the shape of a stripe having a predetermined width, and are arranged in an elongated state in the longitudinal direction L. As the leg hole elastic members 50 extend towards the front end 50A and the rear end 50B positioned outside of the longitudinal direction from the center in the longitudinal direction L, the leg hole elastic members are arranged in a curved shape widening towards the outside of the widthwise direction.

In the back waistline region S2, a fastening member 41 configured to be fastened to the exterior sheet 20 of the front waistline region S1 is provided. The fastening member 41 is joined with the exterior sheet 20. The fastening member 41 is formed of a surface fastener and an adhesive tape.

### (2) Method of manufacturing absorbent article

Next, an example of the method of manufacturing the absorbent article according to the present embodiment is explained. As far as the method that is not described in the present embodiment is concerned, the existing method can be used. Furthermore, the manufacturing method explained below is only an example, and the disposable diaper can also be manufactured by other manufacturing methods. The method of manufacturing the absorbent article includes at least a component forming step, a component loading step, a leg hole forming step, and a cutting step.

In the component forming step, the components configuring the absorbent article are formed. Specifically, for example, the absorber material is laminated and the absorber 30 is molded, and after arranging the continuous stripe-shaped elastic members in a predetermined shape, the elastic members are cut in individual product lengths to form the leg hole elastic members 50.

In the component loading step, components configuring the absorbent article 1, such as the leg hole elastic members 50, other webs such as the web configuring the topsheet, the water-resistive sheet, and the absorber are loaded on the web configuring the exterior sheet 20.

The leg hole forming step is configured to cut the topsheet 10 and the exterior sheet 20 along the concave portion configuring the leg hole region. The leg hole region arranged around the legs of the wearer is thus formed.

In the cutting step, the continuum on which the topsheet 10, the exterior sheet 20, the absorber 30, and the leg hole elastic members 50 are arranged is cut in the size of a single product along the widthwise direction W. The absorbent article 1 is thus manufactured.

### (3) Configuration of a manufacturing apparatus of an absorbent article

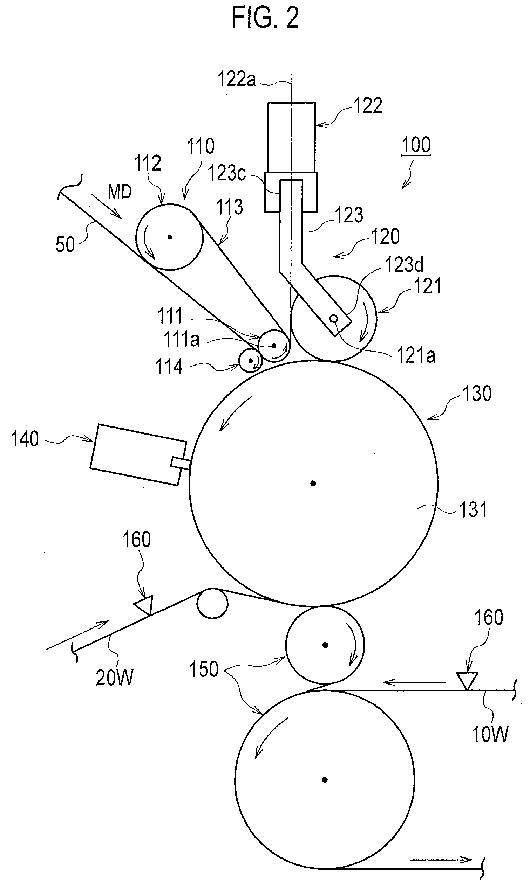

Next, the configuration of a manufacturing apparatus of an absorbent article used in the aforementioned component forming step and the component loading step is explained in detail. Fig. 2 is a side view schematically illustrating a manufacturing apparatus of an absorbent article 100.The manufacturing apparatus of an absorbent article 100 includes a supply mechanism 110, a swinging mechanism 120, a guide mechanism 130, a cutting mechanism 140, an installation mechanism 150, and an adhesive applying mechanism 160. The manufacturing apparatus of an absorbent article 100 is configured to mount the stripe-shaped leg hole elastic members 50 that are conveyed continuously onto a web which is continuous components forming one part of the absorbent article under conveyance.

The supply mechanism 110. is configured to send out the continuous leg hole elastic members 50 towards the swinging mechanism 120. The supply mechanism 110 includes a first rotation mechanism 111, a first roller 112, a belt member 113, and a nip roller 114. The first rotation mechanism 111 is configured to convey the leg hole elastic members in the conveyance direction MD along the outer circumference with a first rotation axis 111a as the turning center. It must be noted that the belt member 113 is arranged on the outer circumference of the first rotation mechanism 111, and the leg hole elastic members are conveyed along the outer circumference of the first rotation mechanism 111 via the belt member 113.

The first roller 112 is arranged towards the upstream side from the first rotation mechanism 111 in the conveyance direction MD. The belt member 113 is an endless belt that traverses between the first rotation mechanism 111 and the first roller 112. The leg hole elastic members 50 are conveyed by the belt member 113 while being in contact on the outer circumference of the belt member 113. The nip roller 114 is arranged opposite the outer circumference of the first rotation mechanism 111. The leg hole elastic members 50 transit between the belt member 113 and the nip roller 114.

The first rotation mechanism 111 and the first roller 112 rotate in the anti-clockwise direction illustrated in Fig. 2, and the belt member 113 moves in the anti-clockwise direction. After being conveyed from the first roller side towards the first rotation mechanism side, the leg hole elastic members separate from the outer circumference of the first rotation mechanism and are conveyed towards the swinging mechanism 120.

The swinging mechanism 120 includes a second rotation mechanism 121, a third rotation mechanism 122, and a connecting arm 123. The second rotation mechanism 121 is configured to convey the leg hole elastic members 50 supplied from the first rotation mechanism 111 along the outer circumference with a second rotation axis 121a as the turning center, and conveys the leg hole elastic members towards a fourth rotation mechanism 131 described later.

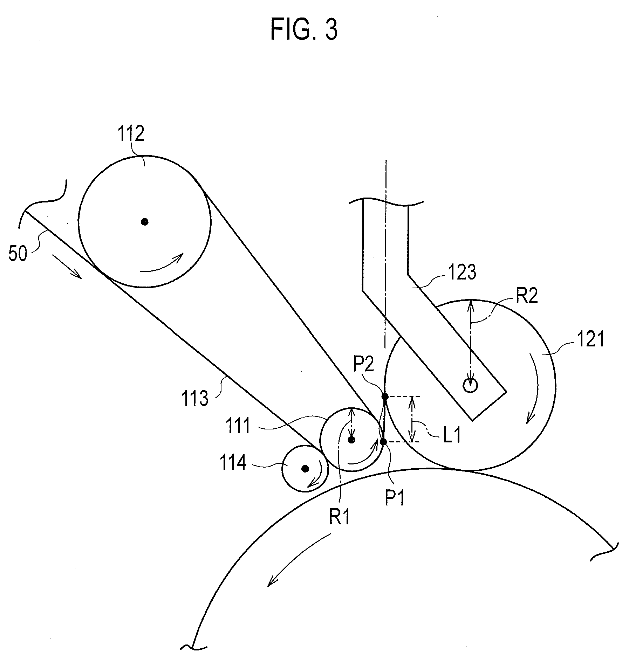

The second rotation mechanism 121 is not driven by a driving means but is configured to rotate due to pulling of the leg hole elastic members 50 by the fourth rotation mechanism 131 positioned downstream of the conveyance direction. The rotating velocity of the fourth rotation mechanism is configured to be faster than the rotating velocity of the first rotation mechanism, and the rotating velocity of the second rotation mechanism 121 is configured to be faster than the rotating velocity of the first rotation mechanism 111. Thus, due to the velocity difference between the first rotation mechanism 111 and the second rotation mechanism 121, the leg hole elastic members 50 are configured to be elongated in the conveyance direction between the separation point P1 where the leg hole elastic members separate from the outer circumference of the first rotation mechanism, and the contact point P2 where the leg hole elastic members come in contact with the second rotation mechanism. The separation point and the contact point are illustrated in Fig. 3.

It must be noted that the contact point P2 in the present embodiment is the contact point that is most upstream of the conveyance direction in the position where the leg hole elastic members are in contact with the outer circumference of the second rotation mechanism, and is the point where the leg hole elastic members guided from the first rotation mechanism side first come in contact with the second rotation mechanism.

The second rotation mechanism 121 is connected to the third rotation mechanism 122 configured to rotate about the third rotation axis 122a, via the connecting arm 123. One end 123c of the connecting arm 123 is connected to the outer circumference of the third rotation mechanism 122, and the other end 123d of the connecting arm 123 is connected to the second rotation axis 121 a of the second rotation mechanism 121. As a result of the rotational driving of the third rotation mechanism 122, the second rotation mechanism 121 swings in the widthwise direction CD orthogonal to the conveyance direction MD. The operation of the second rotation mechanism 121 and the third rotation mechanism 122 is described in detail later. The second rotation mechanism 121 is configured to send out the leg hole elastic members towards the guide mechanism 130 while causing the leg hole elastic members to swing in the widthwise direction CD in a predetermined cycle.

The leg hole elastic members 50 are conveyed towards the guide mechanism 130 while being displaced in the widthwise direction CD, and the leg hole elastic members 50 sent out from the swinging mechanism 120 are arranged in a curved shape on the outer circumference of the guide mechanism 130.

The guide mechanism 130 includes the fourth rotation mechanism 131 configured to send out the elastic members 50 towards the installation mechanism 150 while maintaining the shape of the leg hole elastic members 50 supplied from the swinging mechanism 120, and a driving means (not shown in the figure) configured to perform rotational driving of the fourth rotation mechanism at a velocity faster than that of the first rotation mechanism. The fourth rotation mechanism 131 includes a plurality of retaining members (not shown in the figure), and is configured to convey the leg hole elastic members 50 via the retaining members.

As described above, the second rotation mechanism 121 is not driven by a driving means but is configured to rotate due to being pulled by the leg hole elastic members 50 conveyed by the fourth rotation mechanism 131. The rotating velocity of the second rotation mechanism 121 is almost the same as the rotating velocity of the fourth rotation mechanism 131. Furthermore, the difference in the rotating velocity between the second rotation mechanism 121 and the fourth rotation mechanism 131 is lesser than the difference in the rotating velocity between the second rotation mechanism 121 and the first rotation mechanism 111. According to such a configuration, the leg hole elastic members 50 are not elongated during the course of being guided from the second rotation mechanism 121 to the fourth rotation mechanism 131 with a relatively small rotational velocity difference, and are easily elongated only while guided from the first rotation mechanism 111 to the second rotation mechanism 121 with a relatively high rotational velocity difference. Therefore, by repeatedly elongating the leg hole elastic members 50, the neck-in phenomenon can be prevented.

Furthermore, the send-out direction in which the leg hole elastic members 50 are sent out from the second rotation mechanism 121 towards the fourth rotation mechanism, and the tangential direction of the contact point where the leg hole elastic members come in contact with the fourth rotation mechanism after being sent out from the second rotation mechanism 121 are almost parallel. For example, when the send-out direction and the tangential direction are non-parallel, twisting occurs in the leg hole elastic members 50 during the course of being guided from the second rotation mechanism to the fourth rotation mechanism. However, because the send-out direction of the leg hole elastic members 50 and the tangential direction of the receiving side (fourth rotation mechanism side) are matching, the twisting at the time of receiving and sending can be prevented.

The cutting mechanism 140 is configured to cut the leg hole elastic members 50 being conveyed by the guide mechanism 130 at each retaining member. The leg hole elastic members 50 cut by the cutting mechanism 140 now have the installation length of the leg hole elastic members 50 in each individual product.

The installation mechanism 150 includes a plurality of rollers and is configured to join each component by sandwiching the components between the rollers. Specifically, for example, the installation mechanism intermittently installs the leg hole elastic members 50 cut by the cutting mechanism 140 on an exterior sheet web 20W which is continuous exterior sheet 20 under conveyance.

The adhesive applying mechanism 160 is arranged upstream of the installation mechanism 150, and is a spray-type device configured to apply an adhesive (for example, a hot-melt adhesive) on a web such as the exterior sheet web 20W.

After the leg hole elastic members 50 are bonded onto the exterior sheet, the installation mechanism 150 adheres the absorber 30, to which the adhesive is applied by the adhesive applying mechanism 160, on the topsheet web 10W which is , continuous topsheet.

In this way, a continuum in which the absorbent article is arranged in continuation in the longitudinal direction L is formed.

It must be noted that in the present embodiment, the leg hole elastic members 50 are installed on the exterior sheet web 20W, however, the present embodiment is not limited thereto, and in an absorbent article having side sheets, the leg hole elastic members may be arranged on the web configuring the side sheets, or the leg hole elastic members may be arranged on another web.

### (4) Elongation manner of leg hole elastic members

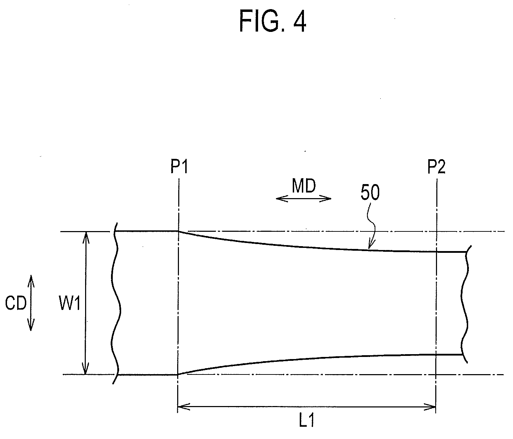

Next, the elongation manner of the leg hole elastic members 50 is explained in detail. The leg hole elastic members are elongated in the conveyance direction while being conveyed from the first rotation mechanism to the second rotation mechanism. Fig. 4 is a plan view schematically illustrating the elongated state of the leg hole elastic members.

The leg hole elastic members 50 are configured such that the elongation rate changes during the course of conveyance by the manufacturing apparatus illustrated in Fig. 2. The elongation rate of the leg hole elastic members 50 conveyed by the supply mechanism 110 is 0 to 20%. A leg hole elastic member having an elongation rate of 0 to 20% is essentially in a state in which it is not elongated, and the width W1 thereof is the width of the elastic member in the non-elongated state. It should be noted that "an elongation rate" of the present invention is defined as a natural state (a state that is not elongated) an elongation rate of 0%, and as elongating to a length of two times an elongation rate of 100%, for example, elongating to a length of 20cm from a length of 10cm. An elongation rate of 20% is to elongated to a length of 12cm from a length of 10cm.

The leg hole elastic member 50 conveyed by the supply mechanism 110 is the leg hole elastic member 50 at the upstream side from the separation point P1, in the conveyance direction, where the leg hole elastic member separates from the outer circumference of the first rotation mechanism. Because the leg hole elastic member 50 essentially does not elongate at the upstream side from the separation point P1, the neck-in phenomenon is less likely to occur before elongation during the time of being guided from the supply mechanism 110 to the swinging mechanism 120. Therefore, for example, as compared to a configuration in which the elastic members are elongated in two stages, the occurrence of the neck-in phenomenon can be prevented.

The elongation rate of the leg hole elastic member 50 that is at the downstream side from the separation point P1 where the leg hole elastic member separates from the outer circumference of the first rotation mechanism, and at the upstream side from the contact point P2 where the leg hole elastic member comes in contact with the outer circumference of the second rotation mechanism is 0% to 20%. The leg hole elastic member 50 is elongated in the conveyance direction, and the width reduces gradually towards the downstream side due to the neck-in phenomenon.

The leg hole elastic member 50 at the contact point P2 with the outer circumference of the second rotation mechanism is in contact with the outer circumference of the second rotation mechanism, and due to the frictional force with the outer circumference of the second rotation mechanism, the state of elongation in the conveyance direction and the state of narrowing in the widthwise direction are maintained. The leg hole elastic member 50 at the downstream side from the contact point P2 with the outer circumference of the second rotation mechanism is conveyed in the conveyance direction in the same state as the contact point P2.

Before and after the leg hole elastic member 50 is elongated, the leg hole elastic member is in contact with the outer circumference of the first rotation mechanism and belt member, and the outer circumference of the second rotation mechanism due to which movement in the widthwise direction is restricted. Therefore, the occurrence of the neck-in phenomenon at the upstream side and downstream side from the distance between the separation point P1 to the contact point P2 can be prevented.

Furthermore, in the manufacturing apparatus of an absorbent article according to the present embodiment, the distance L1 (see Fig. 3 and Fig. 4) between the separation point P1 where the leg hole elastic members 50 separate from the first rotation mechanism 111, and the contact point P2 where the leg hole elastic members 50 come in contact with the second rotation mechanism 121 is configured to be shorter than the turning radius R2 (see Fig. 3) of the second rotation mechanism. Because the distance in which the elastic members are elongated is shorter as compared to the conventional manufacturing apparatus, the occurrence of the neck-in phenomenon can be prevented.

It must be noted that the distance L1 between the separation point P1 where the leg hole elastic members 50 separate from the first rotation mechanism 111, and the contact point P2 where the leg hole elastic members 50 come in contact with the second rotation mechanism 121 may be configured to be shorter than the turning radius R1 of the first rotation mechanism 111, and may be configured to be shorter than the turning radius R1 of the first rotation mechanism 111 as well as shorter than the turning radius R2 of the second rotation mechanism 121.

The turning radius of the first rotation mechanism is the distance between the surface of the first rotation mechanism side of the leg hole elastic member positioned on the outer circumference of the first rotation mechanism (the belt member in the present embodiment) and the first rotation axis. The turning radius of the second rotation mechanism is the distance between the leg hole elastic member that is in contact with the outer circumference of the second rotation mechanism and the second rotation axis.

Furthermore, the conveyance distance of the leg hole elastic members 50 by the second rotation mechanism 121 is configured to be longer than half the outer circumference of the second rotation mechanism 121. By conveying the leg hole elastic members 50, by the second rotation mechanism 121, over a distance that is equal to or more than half the circumference of the second rotation mechanism 121, the contact surface area between the elastic members and the second rotation mechanism becomes larger as compared to the conventional manufacturing apparatus, because of which the frictional force between the leg hole elastic members 50 and the second rotation mechanism 121 can be increased.

For example, if the leg hole elastic member 50 that is in contact with the outer circumference of the second rotation mechanism 121 moves along the outer circumference, the elastic members expand and contract in the widthwise direction while being in contact with the outer circumference because of which there is a further chance of occurrence of the neck-in phenomenon. However, by increasing the frictional force between the outer circumference of the second rotation mechanism and the elastic members, the sliding of the leg hole elastic members on the outer circumference of the second rotation mechanism can be prevented effectively. Therefore, during the course of conveying the leg hole elastic members 50 by the second rotation mechanism 121, the sliding of the leg hole elastic members 50 on the outer circumference of the second rotation mechanism 121 is prevented, and thereby the occurrence of the neck-in phenomenon can be prevented.

In addition, in the first rotation mechanism, the second rotation mechanism 121, and the fourth rotation mechanism configured to convey the leg hole elastic members, it is desired to provide a movement restriction process to restrict the movement in the widthwise direction of the leg hole elastic members conveyed along the outer circumference.

The movement restriction process, for example, is configured so as to increase the frictional force of the outer circumference. The configuration for increasing the coefficient of friction involves the execution of surface processing, such as silicon coating and plasma coating on the outer circumference of each rotation mechanism, as well as the execution of a satin finish along with the surface processing so as to enable restriction of movement of the leg hole elastic members 50 in the widthwise direction by increasing the frictional force.

Furthermore, another movement restriction process is configured to form a hole in the outer circumference that is in contact with the leg hole elastic members, and provide a suction mechanism inside the rotation mechanism so as to suck the leg hole elastic members 50 by the suction mechanism. According to such a configuration, the movement in the widthwise direction of the leg hole elastic members 50 that are conveyed along the outer circumference can be restricted.

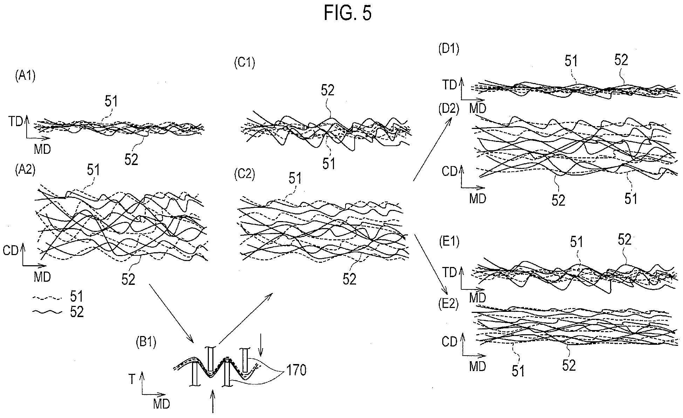

Next, based on Fig. 5, the elongation manner of the leg hole elastic members and the change in the cross-sectional profile due to elongation are explained in detail. Fig. 5 is a cross-sectional view schematically illustrating the elongation manner of the elastic members. Fig. 5 (A1), (B1), (C1), (D1), and (E1) are schematic cross-sectional views of cross-sections of the leg hole elastic members along the conveyance direction MD and the thickness direction TD. Fig. 5 (A2), (C2), (D2), and (E2) are schematic cross-sectional views of cross-sections of the leg hole elastic members along the conveyance direction MD and the widthwise direction CD.

The leg hole elastic members 50 are configured by laminating elastic fibers 51 and non-elastic fibers 52. The leg hole elastic members are already stretched before being supplied to the manufacturing apparatus of an absorbent article, and have elasticity in the conveyance direction.

First of all, the stretching process of the leg hole elastic members is explained. Fig. 5 (A1) and (A2) illustrate the leg hole elastic members before the stretching process, where the elastic fibers 51 and non-elastic fibers 52 are intertwined and laminated.

In the stretching process, as illustrated in Fig. 5 (B1), the leg hole elastic members 50 are sandwiched from both sides of the leg hole elastic members 50 by the convex portions 170 protruding in the leg hole elastic member 50 side. The position of the conveyance direction MD is different for the convex portions 170 positioned on one side of the leg hole elastic members 50 and the convex portions 170 positioned on the other side. As a result of sandwiching the leg hole elastic members 50 by the convex portions 170, the leg hole elastic members 50 are stretched in the thickness direction TD.

Fig. 5 (C1) and (C2) illustrate the leg hole elastic members 50 after the stretching process. The leg hole elastic members 50 after the stretching process are released from the elongation between the convex portions resulting in the contraction of the elastic fibers 51 and the non-elastic fibers 52. At this time, the contractile force in the conveyance direction MD of the elastic fibers 51 is larger than the contractile force in the conveyance direction MD of the non-elastic members 52, because of which the non-elastic fibers 52 take a convex shape in the thickness direction TD and the length of the leg hole elastic members in the thickness direction increases.

Furthermore, because the thickness of the leg hole elastic members 50 increases as a result of stretching of the elastic fibers 51 and the non-elastic fibers 52 in the thickness direction TD, the orientation in the widthwise direction reduces and the orientation in the conveyance direction increases in the plan view. The leg hole elastic member 50 that is at the upstream side from the separation point in the present embodiment is in the state illustrated in Fig. 5 (C1) and (C2).

The leg hole elastic members 50 after the stretching process are elongated in the conveyance direction MD during the course of being guided from the supply mechanism 110 to the swinging mechanism 120, and are in the state illustrated in Fig. 5 (D1) and (D2). Furthermore, the states illustrated in Fig. 5 (E1) and (E2) schematically illustrate the elastic members in the elongated state in the conventional manufacturing apparatus. Fig. 5 (D1) and (D2) illustrate a state in which the neck-in phenomenon is relatively less likely to occur, and Fig. 5. (E1) and (E2) illustrate a state in which the neck-in phenomenon is relatively more likely to occur.

The leg hole elastic members 50 according to the present embodiment are elongated in the conveyance direction in a relatively short distance, and are elongated in the conveyance direction MD in the state when the retention force in the widthwise direction CD is relatively operational. Thus, the orientation state in the widthwise direction CD is maintained for both the elastic fibers 51 and the non-elastic fibers 52, and the orientation state in the thickness direction changes. Therefore, as illustrated in Fig. 5 (D1) and (D2), the length in the widthwise direction of the leg hole elastic members 50 is relatively maintained, and the length in the thickness direction shortens.

On the other hand, the leg hole elastic members elongated by the conventional manufacturing apparatus are elongated in the conveyance direction in a relatively long distance, and are elongated in the conveyance direction MD in the state when the retention force in the widthwise direction CD is relatively non-operational. Thus, the orientation state in the widthwise direction CD changes for both the elastic fibers 51 and the non-elastic fibers 52. Therefore, as illustrated in Fig. 5 (E1) and (E2), the length in the thickness direction of the leg hole elastic members 50 is relatively maintained, and the length in the widthwise direction shortens.

As illustrated in Fig. 5 (E1) and (E2), as the occurrence of the neck-in phenomenon of the leg hole elastic members 50 increases, the length in the widthwise direction of the leg hole elastic members 50 reduces, and stress occurs in the leg hole elastic members 50 along the widthwise direction. The elastic manner manifested in the leg hole elastic members 50 differs in the state when stress occurs along the widthwise direction and the state when the stress does not occur, even if the leg hole elastic members are elongated in the conveyance direction at the same elongation rate. Therefore, due to the occurrence of unwanted stress in the widthwise direction, the desired elastic manner might not be exhibited. However, as illustrated in Fig. 5 (D1) and (D2), if the occurrence of the neck-in phenomenon is less, the occurrence of unwanted stress in the widthwise direction is controlled, and it becomes easy to exhibit the desired elastic manner.

### (5) Swinging Manner of Leg Hole Elastic Members

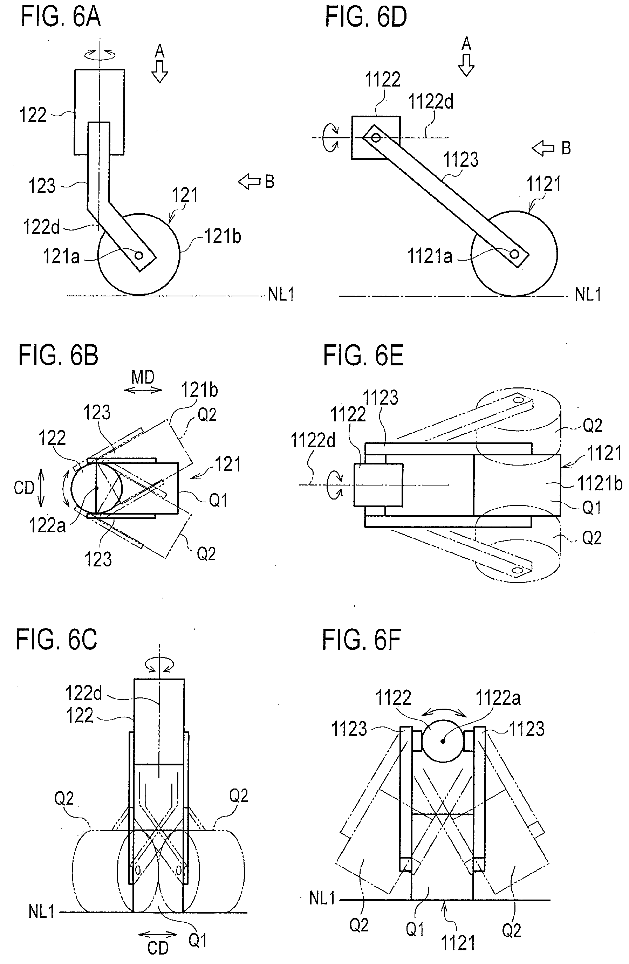

Next, the swinging manner of the leg hole elastic members is explained based on Fig. 6. Fig. 6 schematically illustrates the swinging mechanism portion of the manufacturing apparatus of an absorbent article by which the elastic members are displaced in the widthwise direction. Fig. 6(a) through (c) illustrate the second rotation mechanism 121 and the third rotation mechanism 122, which are a part of the manufacturing apparatus of an absorbent article according to the present embodiment, and by which the leg hole elastic members are displaced in the widthwise direction. Fig. 6(d) through (f) illustrate a part of the conventional manufacturing apparatus of an absorbent article, and for the convenience of explanation, the mechanism configured to convey the elastic members along the outer circumference is explained as a second rotation mechanism 1121, and the mechanism configured to cause the oscillation of the second rotation mechanism 1121 in the widthwise direction is explained as a third rotation mechanism 1122.

First of all, the swinging manner of the leg hole elastic members 50 according to the present embodiment is explained based on Fig. 6 (a) through (c). Fig. 6 (a) is a side view of the absorbent-article manufacturing apparatus, Fig. 6 (b) is the view on arrow A of Fig. 6 (a), and Fig. 6 (c) is the view on arrow B of Fig. 6 (a).

The second rotation mechanism 121 is configured to convey the leg hole elastic members 50 along the outer circumference 121b with the second rotation axis 121 a as the turning center. The second rotation axis 121a runs along the widthwise direction W. As a result of rotation, the second rotation mechanism 121 sequentially feeds the leg hole elastic members 50 in the conveyance direction MD.

The third rotation mechanism 122 is configured to rotate the connecting arm 123 connected to the second rotation axis, with the third rotation axis 122a as the turning center. The leg hole elastic members 50 conveyed by the second rotation mechanism are orthogonal to the direction of arrangement in the fourth rotation mechanism. In a more detailed manner, an axial direction 122d of the third rotation axis 122a is configured to be orthogonal to a contact line NL1 that virtually illustrates the tangential direction of the contact point where the leg hole elastic members 50 conveyed from the second rotation mechanism come in contact with the fourth rotation mechanism, and be orthogonal to the widthwise direction. It must be noted that the contact line NL1 is a tangential line where the elastic members conveyed by the second rotation mechanism come in contact with the web or other conveyance mechanisms.

As a result of the rotation of the third rotation mechanism 122, the second rotation mechanism 121 reciprocates between a center position Q1 that is the swinging center, and an outer end positions Q2 that form both ends in the widthwise direction. The leg hole elastic members 50 are arranged in a waveform within the predetermined range in the widthwise direction CD.

When the second rotation mechanism 121 is at the center position Q1, the leg hole elastic members 50 are arranged in the center of the widthwise direction in a predetermined range. On the other hand, when the second rotation mechanism 121 is at the outer end position Q2, the leg hole elastic members 50 are arranged at the outer end of the widthwise direction in a predetermined range.

The axial direction 122d of the third rotation mechanism is configured to match the tangential direction of the contact point P2 of the second rotation mechanism 121 at the center position Q1. The contact point P2 is the point where the leg hole elastic members 50 supplied from the first rotation mechanism first come in contact with the second rotation mechanism, and is the point where the leg hole elastic members are received by the second rotation mechanism. Because the axial direction 122d of the third rotation mechanism matches the contact point of the center position, the second rotation mechanism swings with the receiving position as the center. Thus, due to the center position and the end positions, the amount of deviation in the distance between the contact point P2 that is the receiving position, and the separation point P1 can be reduced, and the variation in the elongated state due to the occurrence of twisting and the difference in the distance can be prevented.

Furthermore, because the second rotation mechanism 121 according to the present embodiment moves along the contact line NL1, the distance between the second rotation mechanism 121 and the contact line NL1 is constant at the center position Q1 and the outer end positions Q2. That is, as a result of the rotation of the third rotation mechanism 122, the distance between the second rotation mechanism and the contact line does not change, and the second rotation mechanism displaces along the widthwise direction.

For example, if the distance between the leg hole elastic members 50 and the contact line NL1 changes, the conveyance distance of the leg hole elastic members 50 from the time of separation from the second rotation mechanism 121 until the arrival in the fourth rotation mechanism changes, because of which bending and twisting of the leg hole elastic members 50 might occur. However, as the conveyance distance of the leg hole elastic members 50 from the time of separation from the second rotation mechanism until the arrival in the fourth rotation mechanism can be maintained at an almost constant value, the occurrence of twisting in the leg hole elastic members can be prevented.

However, similar to the second rotation mechanism according to the present embodiment, the second rotation mechanism 1121 of the conventional manufacturing apparatus of an absorbent article conveys the leg hole elastic members 50 along an outer circumference 1121b, with a second rotation axis 1121a as the turning center. The second rotation axis 1121 a runs along the widthwise direction W. As a result of rotation, the second rotation mechanism 1121 sequentially feeds the leg hole elastic members 50 in the conveyance direction MD.

The third rotation mechanism 1122 is configured to rotate a connecting arm 1123 connected to the second rotation axis, with the third rotation axis as the turning center. The axial direction 1122d of the third rotation axis is configured to be level with respect to the contact line NL1, and be orthogonal to the widthwise direction.

As a result of the rotation of the third rotation mechanism 1122, the second rotation mechanism 1121 reciprocates between the center position Q1 that is the swinging center, and the outer end positions Q2 that form both ends in the widthwise direction. The leg hole elastic members 50 are arranged in a waveform within the predetermined range in the widthwise direction CD.

When the second rotation mechanism 1121 is at the center position Q1, the leg hole elastic members 50 are arranged in the center of the widthwise direction in a predetermined range. On the other hand, when the second rotation mechanism 1121 is at an outer end position Q2, the leg hole elastic members 50 are arranged in the outer end of the widthwise direction in a predetermined range. In the conventional manufacturing apparatus, the contact line NL1 is configured to be arranged immediately below the second rotation mechanism at the center position.

When the second rotation mechanism 1121 is at an outer end position Q2, the second rotation mechanism 1121 and the contact line NL1 are separated. In this way, if the contact line and the second rotation mechanism are separated at the outer end during oscillation, the amount of deviation in the widthwise direction between the feeding position of the leg hole elastic members 50 in the second rotation mechanism and the contact point on the contact line NL1 increases. If the amount of deviation in the position of the second rotation mechanism configured to feed the leg hole elastic members and the contact line thus increases, it is not possible to realize a fine curved shape. Furthermore, the second rotation mechanism must be caused to swing from the position where the leg hole elastic members 50 must be arranged further up to the outer side of the widthwise direction.

However, as in the case of the manufacturing apparatus of an absorbent article according to the present embodiment, because the distance between the second rotation mechanism 121 and the contact line is constant, the amplitude due to the oscillation of the connecting arm, and the amplitude of the leg hole elastic members is almost matching. Therefore, the swinging angle of the connecting arm at the ends in the widthwise direction of the curved shape can be reduced, and a fine curved shape can be achieved.

According to the manufacturing apparatus of an absorbent article that is thus configured, as a result of the velocity difference in the first rotation mechanism and the second rotation mechanism, stripe-shaped elastic members can be elongated in the conveyance direction from the time of separation from the first rotation mechanism until the arrival in the second rotation mechanism. Because it is possible to elongate the elastic members during the course of being guided from the first rotation mechanism towards the second rotation mechanism, and to convey the elastic members while displacing in the widthwise direction by the second rotation mechanism, stripe-shaped elastic members can be elongated and arranged in a curved shape.

Furthermore, because the distance between the separation point where the elastic members conveyed by the first rotation mechanism separate from the first rotation mechanism, and the contact point where the elastic members come in contact with the second rotation mechanism is shorter than at least either the turning radius of the first rotation mechanism or the turning radius of the second rotation mechanism, and the distance in which the elastic members are elongated is shorter as compared to the conventional manufacturing apparatus, the occurrence of the neck-in phenomenon can be prevented.

Thus, it is possible to provide a manufacturing apparatus of an absorbent article with which it is possible to elongate and arrange stripe-shaped elastic members in a curved state while preventing the neck-in phenomenon.

### (6) Manufacturing apparatus of an absorbent article according to modification

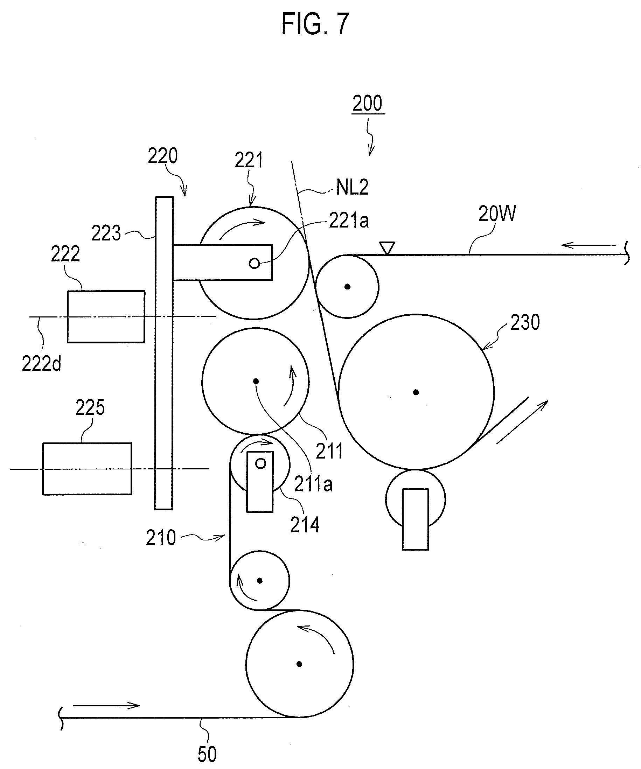

Next, a manufacturing apparatus of an absorbent article 200 according to a modification is explained with reference to Fig. 7. Note that in the explanation of the absorbent-article manufacturing apparatus according to the modification, only the configuration that is different from the manufacturing apparatus of an absorbent article 100 according to the aforementioned embodiment is explained, and the explanation is omitted for configurations that are the same.

The manufacturing apparatus of an absorbent article 200 according to the modification includes a supply mechanism 210, an swinging mechanism 220, and a guide mechanism 230. The manufacturing apparatus of an absorbent article 200 according to the modification is configured to continuously bond the leg hole elastic members 50 onto the continuous exterior sheet web 20W.

The supply mechanism 210 includes a first rotation mechanism 211 configured to rotate with a first rotation axis 211a as the turning center, a plurality of rollers arranged in the conveyance direction from the first rotation mechanism 211, and a nip roller 214 arranged opposite the rollers, and the supply mechanism is configured to feed the leg hole elastic members in continuation towards the swinging mechanism 220.

The swinging mechanism 220 includes a second rotation mechanism 221 configured to convey the leg hole elastic members 50 supplied from the supply mechanism 210 along the outer circumference, a third rotation mechanism 222 configured to displace the second rotation mechanism 221 in the widthwise direction, a servo motor 225 configured to perform oscillatory driving of the third rotation mechanism 222, and a connecting arm 223 configured to connect the second rotation mechanism 221 and the third rotation mechanism 222 and cause the second rotation mechanism to swing in the widthwise direction.

One end of the connecting arm 223 is connected to the third rotation mechanism 222, and the other end of the connecting arm 223 is connected to the second rotation axis 221 a of the second rotation mechanism 221. As a result of the rotational driving of the third rotation mechanism 222, the second rotation mechanism 221 swings in the widthwise direction CD. The leg hole elastic members 50 are sent out towards the guide mechanism 230 while being displaced in the widthwise direction CD, and are loaded on the exterior sheet web 20W conveyed by the guide mechanism 230.

An axial direction 222d of the third rotation axis of the third rotation mechanism 222 is configured to cross a tangential line NL2 where the leg hole elastic members 50 sent out from the second rotation mechanism come in contact with the exterior sheet web 20W, and also be orthogonal to the widthwise direction W. Thus, as a result of the rotation of the third rotation mechanism 222, the second rotation mechanism 221 is displaced along the widthwise direction, and the distance between the second rotation mechanism and the web also changes. However, as the axial direction 222d of the third rotation axis and the tangential line NL2 are almost orthogonal, as compared to the configuration in which the third rotation axis is almost level with the tangential line, similar to the conventional manufacturing apparatus, the amount of deviation in the outer end position can be reduced.

As regards the relationship between the rotating velocity of the first rotation mechanism and the rotating velocity of the second rotation mechanism, there is no particular restriction as long as the rotating velocity of the second rotation mechanism is faster, for example, the velocity of the second rotation mechanism is desired to be around 2.0 to 2.5 times of the velocity of the first rotation mechanism.

### (7) Other embodiments

As described above, although the content of the present invention was disclosed through the embodiments of the present invention, the descriptions and drawings that form a part of this disclosure are not to be considered as limitation to the present invention. Further, various substitutions, examples, or operational techniques shall be apparent to a person skilled in the art on the basis of this disclosure.

As described above, needless to say, the present invention includes various embodiments and the like not described here. Therefore, the technical range of the present invention is to be defined only by the inventive specific matter according to the adequate claims from the above description.

### [Industrial Applicability]

It is possible to provide a manufacturing apparatus of an absorbent article with which it is possible to elongate and arrange stripe-shaped elastic members in a curved state while preventing the neck-in phenomenon.

## Claims

1. A manufacturing apparatus of an absorbent article (1) with which stripe-shaped leg hole elastic members (50) having a predetermined width conveyed continuously are mounted on a web which is components of the absorbent article (1) conveyed continuously, comprising:
a first rotation mechanism (111) configured to convey the elastic members along an outer circumference, with a first rotation axis (111 a) as a turning center; and
a second rotation mechanism (121) configured to convey the plastic members supplied from the first rotation mechanism (111) along the outer circumference while displacing the elastic members (50) in a widthwise direction orthogonal to the conveyance direction of the elastic members, with a second rotation axis (121a) as the turning center, wherein
the distance between a separation point (P1) where the elastic members (50) conveyed by the first rotation mechanism (111) separate from the first rotation mechanism (111), and a contact point (P2) where the elastic members first come in contact with the second rotation mechanism (121) is shorter than the turning radius of at least one of the first rotation mechanism (111) and the second rotation mechanism (121),
wherein the turning radius of the first rotation mechanism (111) is a distance between a surface of a first rotation mechanism side of the leg hole elastic member (50) positioned on an outer circumference of the first rotation mechanism (111) and the first rotation axis (111 a), and the turning radius of the second rotation mechanism (121) is a distance between the leg hole elastic member (50) that is in contact with an outer circumference of the second rotation mechanism (121) and the second rotation axis (121a),
the rotating velocity of the second rotation mechanism (121) is faster than the rotating velocity of the first rotation mechanism (111), and
the elastic members (50) are elongated in the conveyance direction between the separation point (P1) and the contact point (P2), **characterized in that** the second rotation mechanism (121) is connected to a third rotation mechanism (122) configured to rotate about a third rotation axis (122a), and the second rotation mechanism (121) is configured to swing in the widthwise direction as a result of the third rotation mechanism (122),
the elastic members (50) conveyed by the second rotation mechanism (121) are configured to be arranged on the web or to be sent out towards a guide mechanism (130, 230) while being displaced in the widthwise direction, while causing the leg hole elastic members (50) to swing in the widthwise direction in a predetermined cycle, and
the axial direction of the third rotation axis (122a) is configured to cross the tangential direction where the elastic members (50) and the web or the guide member (130, 230) come into contact, and also cross the widthwise direction orthogonally.

2. The manufacturing apparatus of an absorbent article (1) according to claim 1, wherein
the second rotation mechanism (121) is configured to swing between a center position that is the center of a predetermined range in the crossing direction, and end positions that are the ends in the crossing direction in a predetermined range,
the elastic members (50) conveyed by the second rotation mechanism (121) are configured to be arranged on the web or to be sent out towards a guide mechanism (130, 230) while being displaced in the widthwise direction, while causing the leg hole elastic members (50) to swing in the widthwise direction in a predetermined cycle, and
the axial direction of the third rotation axis (122a) matches a tangential direction of the contact point in the center position, and lies orthogonal to the tangential direction where the elastic members (50) and the web or the guide member (130, 230) come into contact.

3. The manufacturing apparatus of an absorbent article (1) according to any one of claim 1 through claim 2, wherein the conveyance distance of the elastic members (50) by the second rotation mechanism (121) is longer than half the outer circumference of the second rotation mechanism (121).

4. The manufacturing apparatus of an absorbent article (1) according to any one of claim 1 through claim 3, wherein the elongation rate in the conveyance direction of the elastic members (50) conveyed by the first rotation mechanism (111) is 0 to 20%.

5. The manufacturing apparatus of an absorbent article (1) according to any one of claim 1 through claim 4, further comprising:
a fourth rotation mechanism (131) configured to convey the elastic members (50) supplied from the second rotation mechanism (121) along the outer circumference, and
a driving means configured to perform rotational driving of the fourth rotation mechanism (131) at a velocity faster than that of the first rotation mechanism (111), wherein
the second rotation mechanism (121) rotates by being pulled by the leg hole elastic members (50) conveyed by the fourth rotation mechanism (131), and
the difference in the rotating velocity between the second rotation mechanism (121) and the fourth rotation mechanism (131) is lesser than the difference in the rotating velocity between the second rotation mechanism (121) and the first rotation mechanism (111).

## Patentansprüche

1. Herstellungsvorrichtung eines saugfähigen Artikels (1), mit der streifenförmige elastische Schenkelöffnungselemente (50) von vorgegebener Breite, die kontinuierlich befördert werden, an einem Gewebe angebracht werden, das Komponenten des kontinuierlich beförderten saugfähigen Artikels (1) ist, umfassen:
einen ersten Drehmechanismus (111), der dazu konfiguriert ist, die elastischen Elemente an einem Außenumfang entlang zu befördern, wobei eine erste Drehachse (111a) ein Drehmittelpunkt ist; und
einen zweiten Drehmechanismus (121), der dazu konfiguriert ist, die elastischen Elemente, die vom ersten Drehmechanismus (111) zugeführt werden, an dem Außenumfang entlang zu befördern, während die elastischen Elemente (50) in einer Breitenrichtung orthogonal zur Beförderungsrichtung der elastischen Elemente verlagert werden, wobei eine zweite Drehachse (121a) der Drehpunkt ist, wobei
der Abstand zwischen einem Trennungspunkt (P1), an dem sich die elastischen Elemente (50), die von dem ersten Drehmechanismus (111) befördert werden, von dem ersten Drehmechanismus (111) trennen, und einem Kontaktpunkt (P2), an dem die elastischen Elemente erstmals in Kontakt mit dem zweiten Drehmechanismus (121) gelangen, kürzer ist als der Wenderadius von wenigstens einem von dem ersten Drehmechanismus (111) und dem zweiten Drehmechanismus (121),
wobei der Wenderadius des ersten Drehmechanismus (111) ein Abstand zwischen einer Fläche einer ersten Drehmechanismusseite des elastischen Schenkelöffnungselements (50), die an einem Außenumfang des ersten Drehmechanismus (111) angeordnet ist, und der ersten Drehachse (111a) ist, und der Wenderadius des zweiten Drehmechanismus (121) ein Abstand zwischen dem elastischen Schenkelöffnungselement (50), das in Kontakt mit
dem Außenumfang des zweiten Drehmechanismus (121) steht, und der zweiten Drehachse (121a) ist,
wobei die Drehgeschwindigkeit des zweiten Drehmechanismus (121) höher als die Drehgeschwindigkeit des ersten Drehmechanismus (111) ist, und
die elastischen Elemente (50) in der Beförderungsrichtung zwischen dem Trennunaspunkt (P1) und dem Kontaktpunkt (P2) verlängert werden, **dadurch gekennzeichnet, dass** der zweite Drehmechanismus (121) mit einem dritten Drehmechanismus (122) verbunden ist, der dazu konfiguriert ist, sich um eine dritte Drehachse (122a) zu drehen, und der zweite Drehmechanismus (121) dazu konfiguriert ist, aufgrund des dritten Drehmechanismus (122) in Breitenrichtung zu schwingen,
die elastischen Elemente (50), die von dem zweiten Drehmechanismus (121) befördert werden, dazu konfiguriert sind, auf dem Gewebe angeordnet zu werden oder zu einem Führungsmechanismus (130, 230) geschickt zu werden, während sie in Breitenrichtung verlagert werden, während sie die elastischen Schenkelöffnungselemente (50) veranlassen, in einem vorgegebenen Zyklus in Breitenrichtung zu schwingen, und
die Axialrichtung der dritten Drehachse (122a) dazu konfiguriert ist, die Tangentialrichtung zu kreuzen, wo die elastischen Elemente (50) und das Gewebe oder das Führungselement (130, 230) in Kontakt gelangen, und außerdem die Breitenrichtung orthogonal zu kreuzen.

2. Herstellungsvorrichtung eines saugfähigen Artikels (1) nach Anspruch 1, wobei
der zweite Drehmechanismus (121) dazu konfiguriert ist, zwischen einer Mittelposition, die die Mitte eines vorgegebenen Bereichs in der Kreuzungsrichtung ist, und Endpositionen zu schwingen, die die Enden in der Kreuzungsrichtung in einem vorgegebenen Bereich sind,
die elastischen Elemente (50), die von dem zweiten Drehmechanismus (121) befördert werden, dazu konfiguriert sind, auf dem Gewebe angeordnet zu werden oder zu einem Führungsmechanismus (130, 230) geschickt zu werden, während sie in Breitenrichtung verlagert werden, während sie die elastischen Schenkelöffnungselemente (50) veranlassen, in einem vorgegebenen Zyklus in Breitenrichtung zu schwingen, und
die Axialrichtung der dritten Drehachse (122a) in der Mittenposition mit einer Tangentialrichtung des Kontaktpunkts übereinstimmt und orthogonal zur Tangentialrichtung liegt, wo die elastischen Elemente (50) und das Gewebe oder das Führungselement (130, 230) in Kontakt gelangen.

3. Herstellungsvorrichtung eines saugfähigen Artikels (1) nach einem der Ansprüche Anspruch 1 bis Anspruch 2, wobei die Förderstrecke der elastischen Elemente (50) durch den zweiten Drehmechanismus (121) länger als die Hälfte des Außenumfangs des zweiten Drehmechanismus (121) ist.

4. Herstellungsvorrichtung eines saugfähigen Artikels (1) nach einem der Ansprüche Anspruch 1 bis Anspruch 3, wobei die Verlängerungsrate in der Förderrichtung der elastischen Elemente (50), die von dem ersten Drehmechanismus (111) befördert werden, 0 bis 20 % beträgt.

5. Herstellungsvorrichtung eines saugfähigen Artikels (1) nach einem der Ansprüche Anspruch 1 bis Anspruch 4, ferner umfassend:
einen vierten Drehmechanismus (131), der dazu konfiguriert ist, die elastischen Elemente (50), die von dem zweiten Drehmechanismus (121) zugeführt werden, an dem Außenumfang entlang zu befördern, und
ein Antriebsmittel, das dazu konfiguriert ist, ein drehendes Antreiben des vierten Drehmechanismus (131) mit einer Geschwindigkeit durchzuführen, die höher als diejenige des ersten Drehmechanismus (111) ist, wobei
der zweiten Drehmechanismus (121) sich dreht, indem er von den elastischen Schenkelöffnungselementen (50) gezogen wird, die von dem vierten Drehmechanismus (131) befördert werden, und die Differenz der Drehgeschwindigkeit zwischen dem zweiten Drehmechanismus (121) und dem vierten Drehmechanismus (131) kleiner als die Differenz der Drehgeschwindigkeit zwischen dem zweiten Drehmechanismus (121) und dem ersten Drehmechanismus (111) ist.

## Revendications

1. Appareil de fabrication d'un article absorbant (1) avec lequel des éléments élastiques à trous de jambe en forme de bande (50) présentant une largeur prédéfinie transportés en continu sont montés sur une toile qui forme des composants de l'article absorbant (1) transportés en continu, comprenant :
un premier mécanisme de rotation (111) conçu pour transporter les éléments élastiques le long d'une circonférence extérieure, avec un premier axe de rotation (111a) en tant que centre de rotation ; et
un deuxième mécanisme de rotation (121) conçu pour transporter les éléments élastiques fournis depuis le premier mécanisme de rotation (111) le long de la circonférence extérieure pendant qu'on déplace les éléments élastiques (50) dans une direction de largeur orthogonale à la direction de transport des éléments élastiques, avec un douzième axe de rotation (121a) en tant que centre de rotation,
la distance entre un point de séparation (P1) où les éléments élastiques (50) transportés par le premier mécanisme de rotation (111) se séparent du premier mécanisme de rotation (111) et un point de contact (P2) où les éléments élastiques viennent d'abord en contact avec le deuxième mécanisme de rotation (121) étant plus courte que le rayon de rotation d'au moins soit le premier mécanisme de rotation (111) soit le deuxième mécanisme de rotation (121),
le rayon de rotation du premier mécanisme de rotation (111) étant une distance entre une surface d'un premier côté du mécanisme de rotation de l'élément élastique de trou de jambe (50) situe sur une circonférence extérieure du premier mécanisme de rotation (111) et le premier axe de rotation (111a) et le rayon de rotation du deuxiéme mécanisme de rotation (121) étant une distance entre l'élément élastique de trou de jambe (50) qui est en contact avec une circonfé- rence extérieure du deuxième mécanisme de rotation (121) et le deuxième axe de rotation (121a), la vitesse de rotation du deuxième mécanisme de rotation (121) étant plus rapide que la vitesse de rotation du premier mécanisme de rotation (111), et
les éléments élastiques (50) étant allongés dans la direction de transport entre le point de séparation (P1) et le point de contact (P2), **caractérisé en ce que**
le deuxième mécanisme de rotation (121) est raccordé à un troisième mécanisme de rotation (122) conçu pour tourneur autour d'un troisième axe de rotation (122a), et le deuxième mécanisme de rotation (121) est conçu pour osciller dans la direction de la largeur du fait du troisième mécanisme de rotation (122),
les éléments élastiques (50) transportés par le deuxième mécanisme de rotation (121) sont conçus pour être disposés sur la toile ou pour être renvoyés vers l'extérieur vers un mécanisme de guidage (130, 230) pendant qu'on les déplace dans la direction de la largeur, pendant qu'on amène les éléments élastiques de trou de jambe (50) à osciller dans la direction de la largeur dans un cycle prédéfini, et
la direction axiale du troisième axe de rotation (122a) est conçue pour traverser la direction tangentielle où les éléments élastiques (50) et la toile ou l'élément de guidage (130, 230) viennent en contact, et traversent aussi la direction de la largeur orthogonalement.

2. Appareil de fabrication d'un article absorbant (1) selon la revendication 1, dans lequel
le deuxième mécanisme de rotation (121) est conçu pour osciller entre une position centrale qui est le centre d'un intervalle prédéfini dans la direction transversale et des positions d'extrémité qui sont les extrémités dans la direction transversale dans un intervalle prédéfini,
les éléments élastiques (50) transportés par le deuxième méchanisme de rotation (121) sont conçus pour être disposés sur la toile ou pour être renvoyés vers l'extérieur vers un mécanisme de guidage (130, 230) pendant leur déplacement dans la direction de la largeur, alors qu'on amène les éléments élastiques de trou de jambe (50) à osciller dans la direction de la largeur dans un cycle prédéfini, et
la direction axiale du troisième axe de rotation (122a) correspond à une direction tangentielle du point de contact dans la position centrale, et repose orthogonalement à la direction tangentielle où les éléments élastiques (50) et la toile ou l'élément de guidage (130, 230) viennent en contact.

3. Appareil de fabrication d'un article absorbant (1) selon l'une quelconque des revendications 1 à 2, dans lequel la distance de transport des éléments élastiques (50) par le douzième mécanisme de rotation (121) est plus grande que la moitié de la circonférence extérieure du deuxième mécanisme de rotation (121).

4. Appareil de fabrication d'un article absorbant (1) selon l'une quelconque des revendications 1 à 3, dans lequel le taux d'allongement dans la direction de transport des éléments élastiques (50) transportés par le premier mécanisme de rotation (111) à de 0 à 20 %.

5. Appareil de fabrication d'un article absorbant (1) selon l'une quelconque des revendications 1 à 4, comprenant en outre :
un quatrième mécanisme de rotation (131) conçu pour transporter les éléments élastiques (50) fournis depuis le deuxième mécanisme de rotation (121) le long de la circonférence extérieure, et
une doyen d'entraînement conçu pour effectuer un entraînement en rotation du quatrième mécanisme de rotation (131) à une vitesse plus rapide que celle du premier mécanisme de rotation (111), dans lequel
le deuxième mécanisme de rota- tion (121) tourne en étant tiré par les éléments élastiques de trou de jambe (50) transportés par le quatrième mécanisme de rotation (131), et
la différence de vitesse de rotation entre le deuxième mécanisme de rotation (121) et le quatrième mécanisme de rotation (131) est plus petite que la différence de vitesse de rotation entre le deuxième mécanisme de rotation (121) et le premier mécanisme de rotation (111).
